# EUROPEAN PATENT SPECIFICATION

(11) **EP 2 028 245 B1**
(45) Date of publication and mention of the grant of the patent: **21.08.2013**
(21) Application number: 08161442.2
(22) Date of filing: 30.07.2008
(51) Int. Cl.: C09D 183/04, A61K 8/02, A61K 8/898

(54) **Film-forming composition**
Filmbildende Zusammensetzung
Composition de formation de film

(30) Priority: 01.08.2007 JP 2007201326
(43) Date of publication of application: 25.02.2009
(73) Proprietor: Shin-Etsu Chemical Co., Ltd., Tokyo 100-0004 (JP)
(72) Inventor: Miyadai, Shinji c/o Silicone-Electronics Mat. Research Center, Annaka-Shi, Gunma 379-0224 (JP); Ono, Ichiro c/o Silicone-Electronics Mat. Research Center, Annaka-Shi, Gunma 379-0224 (JP)
(74) Representative: Hatzmann, Martin

(56) References cited:
- US-A- 5 676 938
- US-A- 6 139 823
- US-A1- 2002 031 488
- US-B1- 6 503 479

## Description

The present invention relates to a film-forming composition, specifically to a film-forming composition which is useful for cosmetics and drugs to protect the skin or hair.

### BACKGROUND OF THE INVENTION

As a film-forming agent to protect surfaces of the skin or hair by providing water repellency to the skin or hair, silicone resins have been widely used. However, a film made from the silicone resin tends to be hard to give twitchy feel to the skin, or the film tends to crack and fall off.

A skin conditioning agent has been presented which comprises 100 parts by weight of a silicone fluid having a viscosity of from 20 to 1,000,000 cSt at 25 °C and 1 to 200 parts by weight of a silicone resin. A film made from the agent, however, is sticky and does not have sufficient adhesion strength to the skin due to relatively large amount of the silicone fluid.

A polysiloxane having a high degree of polymerization of from 3,000 to 20,000 has been used as a film-forming agent for the skin or hair. A film made from the polysiloxane is not sticky, but is soft and has low adhesion strength and abrasion-resistance. To compensate these drawbacks, Japanese Patent Application Laid-Open No.S63-313713 and No.S64-43342 present compositions comprising the aforesaid silicone resin together with the polysiloxane. However, films made from the compositions are still sticky and not satisfactorily abrasion resistant.

Japanese Patent Application Laid-Open No.H04-45155 describes a composition comprising a blend of a specific silicone resin and a specific polysiloxane. A film made from the composition has improved adhesion strength, abrasion resistance and non-stickiness, but still remain rooms for improvement to meet recent high requirements of users.

### SUMMARY OF THE INVENTION

An object of the present invention is to provide a film-forming composition which can form a film which is non-sticky and has an excellent abrasion resistance.

The present inventors have found that the object can be achieved by using a polysiloxane having a specific amount of a C₇₋₃₀ aliphatic hydrocarbon side chains in combination with the aforesaid silicone resin.

The present invention is a film-forming composition in a solution form, comprising
(A) a Silicone resin comprising SiO₂ units and R₃ SiO_{1/2} units, wherein R may be the same with or different from each other and is a C₁₋₆ monovalent hydrocarbon group, with a molar ratio of the R₃SiO_{1/2} units to the SiO₂ units ranging from 0.5 to 1.5, said silicone resin may comprise R₂ SiO and RSiO_{3/2} units, wherein R is as defined above, provided that a total content of the R₃SiO_{1/2} units and the SiO₂ units is at least 80 mole % of all of the units,
(B) an organopolysiloxane represented by the following formula (I), 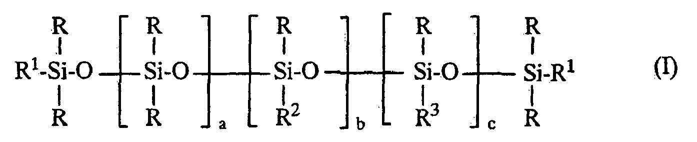 wherein R may be the same with or different from each other and is a C₁₋₆ monovalent hydrocarbon group, R¹ may be the same with or different from each other and is a monovalent group selected from the group consisting of C₁₋₆ monovalent hydrocarbon groups, a hydroxyl group, C₇₋₃₀ monovalent aliphatic hydrocarbon groups, -C₃H₆NH₂ and -C₃H₆NHC₂H₄NH₂, R² may be the same with or different from each other and is a C₇₋₃₀ monovalent aliphatic hydrocarbon group, R³ may be the same with or different from each other and is -C₃H₆NH₂ or -C₃H₆NHC₂H₄NH₂, a is an averaged number ranging from 450 to 2950, b is an averaged number ranging from 50 to 1500, c is an averaged number ranging from 0 to 50, provided that a+b+c ranges from 500 to 3,000, and
(C) an organic solvent,
wherein a weight ratio of component (A) / component(B) ranges from 65/35 to 85/15, and
a content of the component (C) is such that a total concentration of the components (A) and (B) ranges from 0.1 to 50.0 wt% of total weight of the composition.

The above composition can form a non-sticky and abrasion resistant film or coating on a substrate.

### PREFERRED EMBODIMENT OF THE INVENTION

### (A)Silicone resin

In the R₃SiO_{1/2}, R₂SiO, RSiO_{3/2} units, R is a monovalent hydrocarbon group having 1 to 6 carbon atoms. Examples of R include methyl, ethyl, propyl, butyl, pentyl, and hexyl and phenyl groups. A plurality of R' in the silicone resin (A) may be the same with or different from each other. Preferably, 80% or more of R' are methyl groups.

In the component (A), R₃SiO_{1/2} and SiO₂ units are essential units. R₂ SiO and RSiO_{3/2} units may be contained, provided that a total content of R₃SiO_{1/2} and SiO₂ units is 80 mole% or higher, preferably 90 mole% or higher of all the units. A silicone resin having a total content of R₃SiO_{1/2} and SiO₂ units smaller than 80 mole% tends to be less soluble in an organic solvent.

A molar ratio of R₃SiO_{1/2} units to SiO₂ units ranges from 0.5 to 1.5, preferably from 0.7 to 1.2. A silicone resin having a ratio of R₃SiO_{1/2} units to SiO₂ units smaller than 0.5 tends to be less soluble in an organic solvent. On the other hand, a silicone resin having the ratio higher than 1.5 tends to form a sticky film.

### (B) Organopolysiloxane

The organopolysiloxane (B) is represented by the above formula (I). The organopolysiloxane (B) can be prepared by a known method. For example, the one with c being 0 can be prepared by reacting an organohydrogenpolysiloxane represented by the following formula (II) with an C₇₋₃₀ aliphatic hydrocarbon having terminal alkenyl groups to derive Si-R² in the presence of a platinum catalyst , 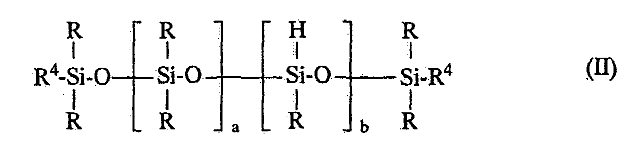 wherein R may be the same with or different from each other and is a C₁₋₆ monovalent hydrocarbon group, R⁴ is a hydrogen atom, a hydroxyl group or a C₁₋₆ monovalent hydrocarbon group, a is an averaged number rangeing from 450 to 2950, b is an averaged number ranging from 50 to 1500, provided that a+b ranges from 500 to 3,000.

Alternatively, the component (B) can be prepared by reacting a polysiloxane of the following formula (III) with a polysiloxane of the formula (IV) or a polysiloxane of the formula (V) in the presence of a basic catalyst such as phosphorous siliconate or potassium siliconate to cause rearrangement of siloxane units.

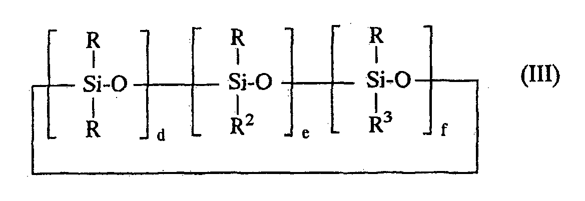

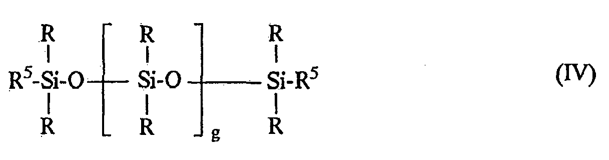

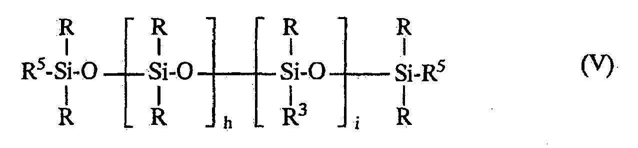

In the above formulas, R is as defied above, R² is a C₇₋₃₀ monovalent aliphatic hydrocarbon group, and R³ is -C₃H₆NH₂ or -C₃H₆NHC₂H₄NH₂, R⁵ is a hydroxyl group, C₁₋₆ monovalent hydrocarbon group, C₇₋₃₀ aliphatic hydrocarbon group, -C₃H₆NH₂ or -C₃H₆NHC₂H₄NH, d is an integer of from 0 to 8, e is an integer of from 1 to 8, f is an integer of from 0 to 8, with d+e+f ranging from 3 to 8, g is an averaged number of from 0 to 3,000 and h is an averaged number of from 1 to 3,000, i is an averaged number of from 1 to 200, with h+i ranging from 1 to 3000.

In the formula (I), R is a monovalent hydrocarbon group having 1 to 6 carbon atoms. Examples of R include methyl, ethyl, propyl, butyl, pentyl, and hexyl and phenyl groups. In the formula (I), a, i.e., the averaged degree of polymerization of R₂SiO units ranges from 450 to 2950, preferably from 600 to 2600. An organopolysiloxane with the degree of polymerization smaller than 450 tends to form a coating too soft to be abrasion resistant. On the other hand an organopolysiloxane with the degree of polymerization larger than 2950 is not miscible with the component (A) and tends to form a film which is sticky and may not be resistant to abrasion.

In the formula (I), R² is a aliphatic hydrocarbon group having 7 to 30 carbon atoms. Examples of R² include alkyl groups such as heptyl, octyl, nonyl, decyl, dodecyl, tetradecyl, hexadecyl, octadecyl, and icosyl groups; and cycloalkyl group such as methylcyclohexyl group. Particularly preferred are aliphatic hydrocarbon groups having 8 to 22 carbon atoms. In the formula (I), b ranges from 50 to 1,500, preferably from 100 to 800. If b is smaller than 50, a coating may not be resistant to abrasion. An organopolysiloxane having b larger than 1,500 tends to be immiscible with the component (A), so that a film may not be uniform, non-sticky or abrasion-resistant.

In the formula (I), R³ is -C₃H₆NH₂ or -C₃H₆NHC₂H₄NH₂. By the amino groups, adhesion strength of a coating can be increased. However, too many amino groups may make a coating sticky. In the formula (I), c therefore ranges from 0 to 50.

In the formula (I), the sum of a, b and c on average ranges from 500 to 3,000, preferably from 800 to 2,500. If the sum is smaller than the aforesaid lower limit, a coating may not be hard enough to be abrasion resistant. An organopolysiloxane having the sum larger than the aforesaid upper limit tends to be immiscible with the component (A), so that a film may not be uniform, non-sticky or abrasion-resistant.

A blending ratio of the component (A) to the component (B) ranges from 65/35 to 85/15, preferably from 70/30 to 80/20. If the ratio exceeds the aforesaid upper limit, a film may not be satisfactorily abrasion resistant. If the ratio is below the aforesaid lower limit, a coating tends to be sticky.

### (C) Organic solvent

The component (C) is used as a solvent for the components (A) and (B). Examples of the organic solvent include volatile siloxane, saturated aliphatic hydrocarbon, saturated cyclic hydrocarbons, aromatic hydrocarbons, chlorohydrocarbons, chlorofluorohydrocarbons, and alcohols. Particularly preferred are volatile organosiloxanes and light liquid isoparaffins having a boiling point at atmospheric pressure of from 100 to 250 °C.

Examples of the volatile organosiloxane include cyclic siloxanes such as hexamethylcyclotrisiloxane, octamethylcyclotetrasiloxane, decamethylcyclopentasiloxane, dodecamethylcyclohexasiloxane, trimethyltriethylcyclotrisiloxane,hexaethyltricyclosiloxane, diethyltetramethylcyclotrisiloxane,dimethyltetraethylcyclotrisilox ane,diethylhexamethylcyclotetrasiloxane,tetraethyltetramethylcyclo tetrasiloxane;linear siloxanes such as hexamethyldisiloxane, octamethyltrisiloxane,decamethyltetrasiloxane, dodecamethylpentasiloxane, hexaethyldisiloxane, octaethyltrisiloxane;branched siloxanes such as methyltris(trimethylsiloxy)silane, phenyltris(trimethylsiloxy)silane.

Examples of light liquid isoparaffin include isoparaffins having 8 to 16 carbon atoms, and those commercial available under the trade names of Isopars C,E,G, H,L,and M supplied by Exxon Mobile Co., IP Solvents 1016,1620, and 2028 supplied by Idemitsu Petrochemical Co., Ltd., Markasol R supplied by Maruzen Petrochemical Co., Nisseki Isosols 300 and 400 supplied by Nippon Petrochemical Co., and Shellsol 71 supplied by Shell Chemical Co. Among these isoparaffins qrepreferred, and isododecane is particularly preferred.

The component (C) is incorporated in the composition in such an amount that a total content of the components (A) and (B) ranges from 0.1 to 50 wt%, preferably from 1 to 30 wt%, of total weight of the composition. If the total content is lower than the aforesaid lower limit, a uniform coating may not be obtained. If the total content exceeds the aforesaid upper limit, a film may be too thick for the component (C) to fully evaporate, making the film sticky.

In addition to the aforesaid components (A) to (C), the present composition can contain commonly used additives such as antioxidants in an amount not to adversely affect the composition.

The present composition can be prepared by dissolving the components (A) and (B), and optional components, if desired, in the component (C) at room temperature.

### EXAMPLES

The present invention will be explained with reference to the following Examples, but not limited thereto.

### Examples 1-14 and Comparative Examples 1-6

Film-forming compositions were prepared by dissolving components (A) and (B) according to the recipes as shown in Tables 1 and 2 in isododecane sold under the trade name "Markasol R" from Maruzen Petrochemical Co. From each of the compositions, two films were prepared by the following methods.

### Heat and dry method

Ten grams of the composition was placed in an aluminum plate having an internal diameter of 60 mm and a depth of 10 mm, which was then placed in a hot-air circulation type oven at 150 °C. After 3 hours, the plate was taken out from the oven and cooled to room temperature.

### Apply on the skin method

On the face skin, 0.1 g of the composition was applied uniformly and left stood for 1 hour.

### Evaluation

The films were evaluated according to the methods described below. The results are as shown in Table 2, wherein "Ex." stands for Example, "Comp.Ex." for Comparative Example, "Conc." for concentration, "Trans." for transparency, "Non-Stick." for non-stickiness, and "Ab.Res." for abrasion resistance.

### (1) Transparency

Transparency of the films were evaluated by visual observation and rated according to the following criteria.
A: Transparent
B: Hardly cloudy
C: A little cloudy
D: Cloudy

### (2) Non-Stickiness

A surface of the films were touched by a finger and evaluated for non-stickiness and slickness, and rated according to the following criteria.
A: Non-sticky and slick
B: Hardly sticky and a little slick
C: A little sticky and hardly slick
D: Sticky and non-slick

### (3) Abrasion resistance

A surface of the films were scrubbed by a finger nail and rated according to the following criteria.
A: Not damaged at all
B: Hardly damaged
C: A little damaged
D: Damaged

### Materials used

### (A) Silicone resin

Silicone resin I consisting of (CH₃)₃SiO_{1/2} units and SiO₂ units with(CH₃)₃SiO_{1/2}:SiO₂=0.85:1;

Silicone resin II consisting of (CH₃)3SiO_{1/2} units and SiO₂ units with (CH₃)₃SiO_{1/2}:SiO₂=0.70:1

### (B) Organopolysiloxane

Organopolysiloxanes represented by the following formula (VI) with the R², R³, and the averaged numbers as shown in Table 1 were used.

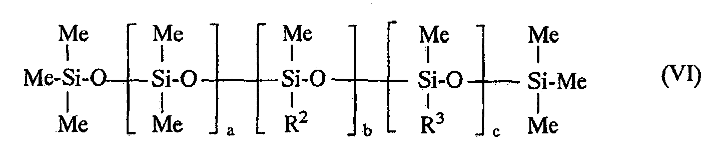

**Table 1**

| (B) Organopolysiloxane | Parameters | | | | | |
|---|---|---|---|---|---|---|
| | R² | R³ | a | b | c | a+b+c |
| 1 | -C₁₆H₃₃ | -- | 700 | 100 | 0 | 800 |
| 2 | -C₁₆H₃₃ | -- | 2400 | 100 | 0 | 2500 |
| 3 | -C₁₆H₃₃ | -- | 700 | 700 | 0 | 1400 |
| 4 | -C ₈H₁₇ | -- | 700 | 100 | 0 | 800 |
| 5 | -C₂₂H₄₅ | -- | 700 | 100 | 0 | 800 |
| 6 | -C₁₆H₃₃ | -C₃H₆NH₂ | 700 | 100 | 10 | 800 |
| 7 | -C₁₆H₃₃ | -- | 300 | 100 | 0 | 400 |
| 8 | -C₁₆H₃₃ | -- | 3000 | 100 | 0 | 3100 |
| 9 | -C₁₆H₃₃ | -C₃H₆NH₂ | 700 | 100 | 100 | 900 |
| 10 | -- | -- | 800 | 0 | 0 | 800 |
| 11 | -C₁₆H₃₃ | | 700 | 30 | 0 | 730 |
| 12 | -C₁₆H₃₃ | | 700 | 1600 | 0 | 2300 |

**Table 2**

| | Composition | | | | Film Properties | | | | | |
|---|---|---|---|---|---|---|---|---|---|---|
| | (A) | (B) | (A)/(B) | Conc. | Heat and Dry | | | Apply on the skin | | |
| | | | wt. ratio | wt% | Trans. | Non-stick. | Ab.Res. | Trans. | Non-Stick. | Ab.Res. |
| Ex.1 | I | 1 | 75/25 | 10 | A | A | A | A | A | A |
| Ex.2 | I | 1 | 85/15 | 10 | A | A | A | A | B | A |
| Ex.3 | I | 1 | 65/35 | 10 | A | A | A | A | A | B |
| Ex.4 | I | 2 | 75/25 | 10 | A | B | A | A | B | A |
| Ex.5 | I | 3 | 75/25 | 10 | B | A | A | B | A | A |
| Ex.6 | I | 4 | 75/25 | 10 | A | A | A | A | A | A |
| Ex.7 | I | 5 | 75/25 | 10 | A | A | A | A | A | A |
| Ex.8 | I | 6 | 75/25 | 10 | A | A | A | A | A | A |
| Ex.9 | II | 1 | 75/25 | 10 | A | A | A | A | A | A |
| Ex.10 | II | 2 | 75/25 | 10 | A | A | A | A | B | A |
| Ex.11 | II | 3 | 75/25 | 10 | B | A | A | B | A | A |
| Ex.12 | II | 4 | 75/25 | 10 | A | A | A | A | A | A |
| Ex.13 | II | 5 | 75/25 | 10 | A | A | A | A | A | A |
| Ex.14 | II | 6 | 75/25 | 10 | A | A | A | A | A | A |
| Comp.Ex.1 | I | 7 | 75/25 | 10 | A | D | D | A | D | D |
| Comp.Ex.2 | I | 8 | 75/25 | 10 | A | C | C | A | C | C |
| Comp.Ex.3 | I | 9 | 75/25 | 10 | A | D | C | A | D | C |
| Comp.Ex.4 | I | 10 | 75/25 | 10 | A | B | C | A | B | C |
| Comp.Ex.5 | I | 11 | 75/25 | 10 | A | B | C | A | B | C |
| Comp.Ex.6 | I | 12 | 75/25 | 10 | D | C | B | D | C | B |

### INDUSTRIAL APPLICABILITY

The composition of the present invention can form a non-sticky and abrasion resistant film and therefore useful for cosmetics or drugs to protect surface of the skin or hair.

## Claims

1. A film-forming composition in a solution form, comprising
(A) a Silicone resin comprising SiO₂ units and R₃SiO_{1/2} units, wherein R may be the same with or different from each other and is a C₁₋₆ monovalent hydrocarbon group, with a molar ratio of the R₃SiO_{1/2} units to the SiO₂ units ranging from 0.5 to 1.5, said silicone resin may comprise R₂SiO and RSiO_{3/2} units, wherein R is as defined above, provided that a total content of the R₃SiO_{1/2} units and the SiO₂ units is at least 80 mole % of all of the units,
(B) an organopolysiloxane represented by the following formula (I), 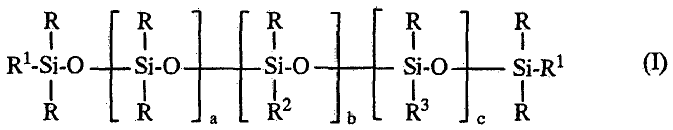 wherein R may be the same with or different from each other and is a C₁₋₆ monovalent hydrocarbon group, R¹ may be the same with or different from each other and is a monovalent group selected from the group consisting of C₁₋₆ monovalent hydrocarbon groups, a hydroxyl group, C₇₋₃₀ monovalent aliphatic hydrocarbon groups, -C₃ H₆ NH₂ and -C₃ H₆ NHC₂ H₄ NH₂, R² may be the same with or different from each other and is a C₇₋₃₀ monovalent aliphatic hydrocarbon group, R³ may be the same with or different from each other and is -C₃ H₆ NH₂ or -C₃ H₆ NHC₂ H₄ NH₂, a is an averaged number ranging from 450 to 2950, b is an averaged number ranging from 50 to 1500, c is an averaged number ranging from 0 to 50, provided that a+b+c ranges from 500 to 3,000, and
(C) an organic solvent,
wherein a weight ratio of component (A) / component(B) ranges from 65/35 to 85/15, and
a content of the component (C) is such that a total concentration of the components (A) and (B) ranges from 0.1 to 50.0 wt% of total weight of the composition.

2. The film-forming composition according to claim 1, wherein the organic solvent (C) is a volatile siloxane and/or light liquid isoparaffin having a boiling point of from 100 to 250 °C at atmospheric pressure.

## Patentansprüche

1. Filmbildende Zusammensetzung in Form einer Lösung, umfassend
(A) ein Siliconharz, umfassend SiO₂-Einheiten und R₃SiO_{1/2}-Einheiten, wobei R gleich oder verschieden voneinander sein kann und eine C₁₋₆ einwertige Kohlenwasserstoffgruppe ist, mit einem molaren Verhältnis der R₃SiO_{1/2}-Einheiten zu den SiO₂-Einheiten im Bereich von 0,5 bis 1,5, wobei das Silikonharz R₂SiO₂-Einheiten und R₂SiO_{3/2}-Einheiten umfassen kann, wobei R wie oben definiert ist, vorausgesetzt dass ein Gesamtgehalt der R₃SiO_{1/2}-Einheiten und der SiO₂-Einheiten mindestens 80 Molprozent aller Einheiten ausmacht,
(B) ein Organopolysiloxan, dargestellt durch die folgende Formel (I), 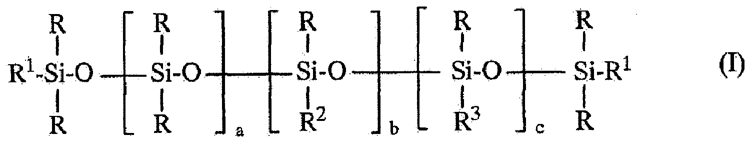 wobei R gleich oder verschieden voneinander sein kann und eine C₁₋₆ einwertige Kohlenwasserstoffgruppe darstellt, R¹ gleich oder verschieden voneinander sein kann und eine einwertige Gruppe ist, ausgewählt aus der Gruppe bestehend aus C₁₋₆ einwertigen Kohlenwasserstoffgruppen, einer Hydroxylgruppe, C₇₋₃₀ einwertigen aliphatischen Kohlenwasserstoffgruppen, -C₃H₆NH₂ und-C₃H₆NHC₂H₄NH₂, R² gleich oder verschieden voneinander sein kann und eine C₇₋₃₀ einwertige aliphatische Kohlenwasserstoffgruppe ist, R³ gleich oder verschieden voneinander sein kann und -C₃H₆NH₂ oder -C₃H₆NHC₂H₄NH₂ ist, a eine durchschnittliche Zahl im Bereich von 450 bis 2950 ist, b eine durchschnittlichen Zahl im Bereich von 50 bis 1500 ist, c eine durchschnittliche Zahl im Bereich von 0 bis 50 ist, vorausgesetzt dass a + b + c im Bereich von 500 bis 3000 liegt, und
(C) ein organisches Lösungsmittel,
wobei ein Gewichtsverhältnis der Komponente (A) / Komponente (B) im Bereich von 65/35 bis 85/15 liegt, und
ein Gehalt der Komponente (C) derart ist, dass eine Gesamtkonzentration der Komponenten (A) und (B) im Bereich von 0,1 bis 50,0 Gewichtsprozent des Gesamtgewichts der Zusammensetzung liegt.

2. Filmbildende Zusammensetzung nach Anspruch 1, wobei das organische Lösungsmittel (C) ein flüchtiges Siloxan und/oder leichtes flüssiges Isoparaffin mit einem Siedepunkt von 100 bis 250 °C bei Normaldruck ist.

## Revendications

1. Composition formant un film dans une forme de solution, comprenant
(A) une résine de silicone comprenant des unités SiO₂ et des unités R₃SiO_{1/2}, où les R peuvent être identiques ou différents les uns des autres, et sont un groupe hydrocarboné monovalent en C₁₋₆, avec un rapport molaire des unités R₃SiO_{1/2} aux unités SiO₂ de 0,5 à 1,5, ladite résine de silicone peut comprendre des unités R₂SiO et RSiO_{3/2}, où R est comme défini ci-dessus, à condition qu'une teneur totale des unités R₃SiO_{1/2} et des unités SiO₂ est d'au moins 80 % en mol de toutes les unités,
(B) un organopolysiloxane représenté par la formule (I)
suivante, 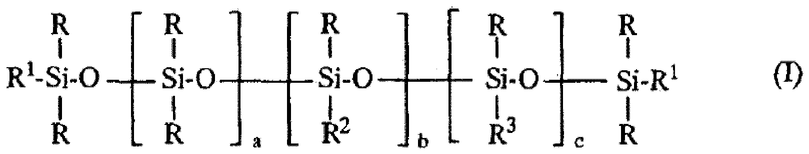 où les R peuvent être identiques ou différents les uns des autres et sont un groupe hydrocarboné monovalent en C₁₋₆, les R¹ peuvent être identiques ou différents les uns des autres et sont un groupe monovalent choisi dans le groupe constitué de groupes hydrocarbonés monovalents en C₁₋₆, d'un groupe hydroxyle, de groupes hydrocarbonés aliphatiques monovalents en C₇₋₃₀, de -C₃H₆NH₂ et de -C₃H₆NHC₂H₄NH₂, les R² peuvent être identiques ou différents les uns des autres et sont un groupe hydrocarboné aliphatique monovalent en C₇₋₃₀, les R³ peuvent être identiques ou différents les uns des autres et sont -C₃H₆NH₂ ou -C₃H₆NHC₂H₄NH₂, a est un nombre moyenné de 450 à 2 950, b est un nombre moyenné de 50 à 1 500, c est un nombre moyenné de 0 à 50, à condition que a+b+c est de 500 à 3 000, et
(C) un solvant organique,
dans laquelle un rapport massique du constituant (A)/constituant (B) est de 65/35 à 85/15, et
une teneur du constituant (C) est telle qu'une concentration totale des constituants (A) et (B) est de 0,1 à 50,0 % en masse de la masse totale de la composition.

2. Composition formant un film selon la revendication 1, dans laquelle le solvant organique (C) est un siloxane volatil et/ou une isoparaffine liquide légère ayant un point d'ébullition de 100 à 250°C à pression atmosphérique.
